# EUROPEAN PATENT SPECIFICATION

(11) **EP 3 703 062 B1**
(45) Date of publication and mention of the grant of the patent: **30.08.2023**
(21) Application number: 20151364.5
(22) Date of filing: 13.01.2020
(51) Int. Cl.: G16C 20/50, G16C 20/40

(54) **COMPOUND SEARCH METHOD, COMPOUND SEARCH DEVICE, AND COMPOUND SEARCH PROGRAM**
VERBINDUNGSSUCHVERFAHREN, VERBINDUNGSSUCHVORRICHTUNG UND VERBINDUNGSSUCHPROGRAMM
PROCÉDÉ DE RECHERCHE DE COMPOSÉS, DISPOSITIF DE RECHERCHE DE COMPOSÉS ET PROGRAMME DE RECHERCHE DE COMPOSÉS

(30) Priority: 27.02.2019 JP 2019033973
(43) Date of publication of application: 02.09.2020
(73) Proprietor: FUJITSU LIMITED, Kanagawa 211-8588 (JP)
(72) Inventor: MITSUI, Takashi, Kawasaki-shi, Kanagawa 211-8588 (JP)
(74) Representative: Haseltine Lake Kempner LLP

(56) References cited:
- WO-A2-2010/079345
- SUSANNA MONTI ET AL: "Exploring the conformational and reactive dynamics of biomolecules in solution using an extended version of the glycine reactive force field", PHYSICAL CHEMISTRY CHEMICAL PHYSICS, vol. 115, no. 36, 1 September 2013 (2013-09-01), pages 15062-15077, XP055706093, ISSN: 1463-9076, DOI: 10.1039/c3cp51931g
- HARPOLE TYLER J ET AL: "Conformational landscapes of membrane proteins delineated by enhanced sampling molecular dynamics simulations", BBA - BIOMEMBRANES, ELSEVIER, AMSTERDAM, NL, vol. 1860, no. 4, 4 November 2017 (2017-11-04), pages 909-926, XP085349965, ISSN: 0005-2736, DOI: 10.1016/J.BBAMEM.2017.10.033
- THOMAS STEINBRECHER ET AL: "Free energy calculations on the binding of novel thiolactomycin derivatives tofatty acid synthase I", BIOORGANIC & MEDICINAL CHEMISTRY : A TETRAHEDRON PUBLICATION FOR THE RAPID DISSEMINATION OF FULL ORIGINAL RESEARCH PAPERS AND CRITICAL REVIEWS ON BIOMOLECULAR CHEMISTRY, MEDICINAL CHEMISTRY AND RELATED DISCIPLINES, ELSEVIER, NL, vol. 20, no. 11, 7 April 2012 (2012-04-07) , pages 3446-3453, XP028423300, ISSN: 0968-0896, DOI: 10.1016/J.BMC.2012.04.019 [retrieved on 2012-04-16]

## Description

### FIELD

The embodiments discussed herein are related to a compound search method, a compound search device, and a compound search program.

### BACKGROUND

In a case where a target molecule such as protein has a functional site (an active site) related to a disease, drug discovery having the target molecule needs to design a ligand that stably binds to the functional site of the target molecule. As the ligand stably binds to the target molecule, the functional site of the target molecule is blocked, for example. As a result, the function related to the disease of the target molecule is reduced.

Ligand design methods that utilize structural information about a target molecule are roughly divided into two types.

One is a method for designing a ligand on the basis of the three-dimensional structure of the target molecule, and is called structure-based drug design (SBDD). By this method, the optimum structure of a ligand is normally searched for with respect to the fixed three-dimensional structure of the target molecule. However, the actual three-dimensional structure of the target molecule is fluctuating in vivo. Further, it is known that the three-dimensional structure of the active site of the target molecule changes depending on the structure of a ligand. Furthermore, a ligand designed based on the static three-dimensional structure of the target molecule, and the target molecule may or may not form a stable bonded structure even in a dynamic environment.

Another ligand design method is a method for designing a ligand by combining or growing fragment molecules that easily bind to an active site. This ligand design method is called fragment-based drug design (FBDD). By this method, the stable three-dimensional structure of the target molecule changes due to a difference in the structure of the fragment molecule that binds to the active site. As a result, a designed ligand might not appropriately bind to the target molecule in practice.

Therefore, there is a demand for a ligand (compound) design method that takes into consideration changes in the three-dimensional structure of the target molecule.

Japanese National Publication of International Patent Application No. 2002-533477 is disclosed as related art. .S. Monti et al, PHYSICAL CHEMISTRY CHEMICAL PHYSICS, vol. 115, no. 36, 1 September 2013 (2013-09-01), pages 15062-15077, discloses exploring the conformational and reactive dynamics of biomolecules in solution.

It is desirable to provide a compound search method, a compound search device, and a compound search program for searching for a compound having a strong interaction with a target molecule, taking into consideration changes in the three-dimensional structure of the target molecule.

### SUM MARY

The present invention is defined by the appended independent claims, to which reference should now be made. Specific embodiments are defined in the dependent claims.

According to an embodiment of one aspect of the invention, it may be possible to provide a compound search method for searching for a compound having a strong interaction with a target molecule, taking into consideration changes in the three-dimensional structure of the target molecule.

Further, according to another embodiment of one aspect of the invention, it is possible to provide a compound search device that searches for a compound having a strong interaction with a target molecule, taking into consideration changes in the three-dimensional structure of the target molecule.

Further, according to yet another embodiment of one aspect of the invention, it is possible to provide a compound search program for searching for a compound having a strong interaction with a target molecule, taking into consideration changes in the three-dimensional structure of the target molecule.

### BRIEF DESCRIPTION OF DRAWINGS

The invention is described, by way of example only, with reference to the
following drawings, in which:
FIG. 1A is a schematic diagram (first diagram) for explaining a method of sequentially creating compounds compatible with the binding site of a target molecule in a dynamic environment;
FIG. 1B is a schematic diagram (second diagram) for explaining the method of sequentially creating compounds compatible with the binding site of a target molecule in a dynamic environment;
FIG. 1C is a schematic diagram (third diagram) for explaining the method of sequentially creating compounds compatible with the binding site of a target molecule in a dynamic environment;
FIG. 2 is a flowchart of an example of a compound search method;
FIG. 3 is a flowchart of an example of a growth step;
FIG. 4A is a schematic diagram (first diagram) for explaining an example of a growth step;
FIG. 4B is a schematic diagram (second diagram) for explaining an example of a growth step;
FIG. 4C is a schematic diagram (third diagram) for explaining an example of a growth step;
FIG. 4D is a schematic diagram (fourth diagram) for explaining an example of a growth step;
FIG. 4E is a schematic diagram (fifth diagram) for explaining an example of a growth step;
FIG. 5 is a flowchart of an example of an extraction step;
FIG. 6 is a diagram for explaining an example of the numbers of times the respective molecules obtained as a result of the growth step have been generated;
FIG. 7A illustrates a plurality of grown molecules obtained as a result of an example of the growth step;
FIG. 7B is a diagram illustrating an example of an atom density distribution;
FIG. 8 is a diagram for explaining an example of a method for creating a molecule;
FIG. 9A is a diagram (first diagram) for explaining an actual example of the growth step;
FIG. 9B is a diagram (second diagram) for explaining an actual example of the growth step;
FIG. 9C is a diagram (third diagram) for explaining an actual example of the growth step;
FIG. 10 is a hardware configuration diagram of an example of a compound search device disclosed herein;
FIG. 11 is a hardware configuration diagram of another example of the disclosed compound search device; and
FIG. 12 is a hardware configuration diagram of yet another example of the disclosed compound search device.

### DESCRIPTION OF EMBODIMENTS

Drug discovery refers to the process of designing a medicinal drug. Drug discovery is performed in the following order, for example.
(1) Target molecule determination
(2) Search for lead compounds and the like
(3) Physiological action test
(4) Safety and toxicity tests

In searching for lead compounds and the like (lead compounds and compounds derived therefrom), it is important to accurately evaluate the interaction between each molecule of a large number of drug candidate molecules and a target molecule.

The process of designing a medicinal drug using a computer is sometimes called in silico drug discovery. The in silico drug discovery technology can be used in general drug discovery. Particularly, the use of the in silico drug discovery technology in searching for lead compounds and the like is useful for shortening the new drug development period and increasing the probability of new drug development, for example.

The technology disclosed herein may be used in searching for lead compounds and the like that are expected to have high pharmacological activity, for example.

### (Compound Search Method)

A compound search method of the present application is a compound search method for searching for a compound having a strong interaction with a target molecule.

The compound search method includes a growth step, for example.

The compound search method includes an extraction step, for example.

In the growth step, molecular dynamics calculation using a reactive force field is performed, and atoms are bonded to the base fragment molecule at the binding site (binding pocket) of the target molecule, so that the base fragment molecule is grown, and a grown molecule is obtained.

In the extraction step, a molecule created with the use of the appearance frequency in the step of growth of each of the chemical structures of a plurality of grown molecules obtained in a plurality of growth steps, and an atom density distribution obtained by superimposing the grown molecules obtained in the plurality of growth steps is used, to extract a candidate compound that is a candidate for a compound.

In both SBDD and FBDD, which are ligand design methods, changes in the three-dimensional structure of protein are not taken into consideration, and therefore, a bonded structure of the target molecule that is protein and a drug candidate molecule that is a designed ligand is not sufficiently stable many cases.

To counter this, the present inventor came up with the idea of sequentially creating compounds compatible with the binding site of the target molecule in a dynamic environment. This concept is now described.

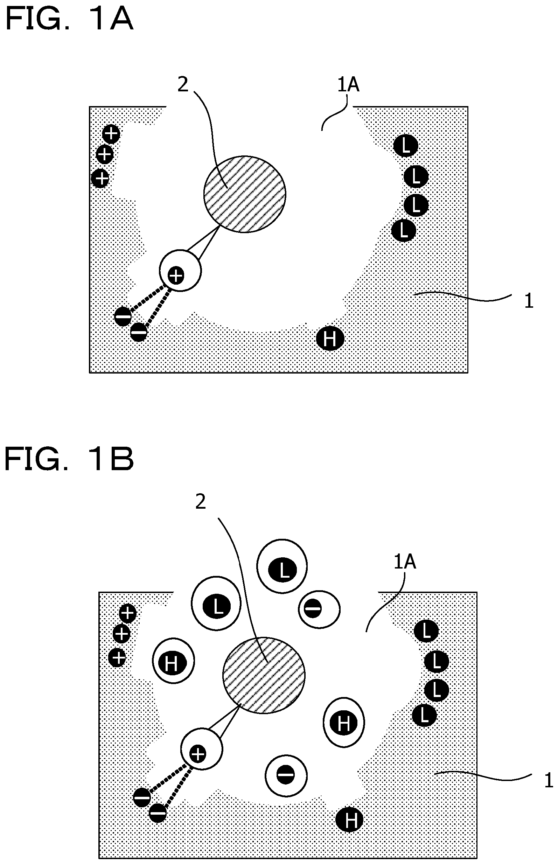

First, a base fragment molecule 2 is placed at the binding site 1A of a target molecule 1 (FIG. 1A).

Atoms or groups of atoms are then placed at the binding site 1A as appropriate (FIG. 1B). Here, in FIG. 1B, "H" represents a hydrophilic atom or a group of hydrophilic atoms, "L" represents a lipophilic atom or a group lipophilic atoms, "+" represents a positively charged atom or a group of positively charged atoms, "-" represents a negatively charged atom or a group of negatively charged atoms. In that state, molecular dynamics calculation using a reactive force field is then performed. Here, in the molecular force field that is a force field normally used in molecular dynamics calculation, generation or cleavage of covalent bonds is not taken into consideration. In a reactive force field, on the other hand, generation or cleavage of covalent bonds is taken into consideration. Therefore, in the molecular dynamics calculation, it is possible to bond the base fragment molecule 2 to an atom or a group of atoms, using a reactive force field.

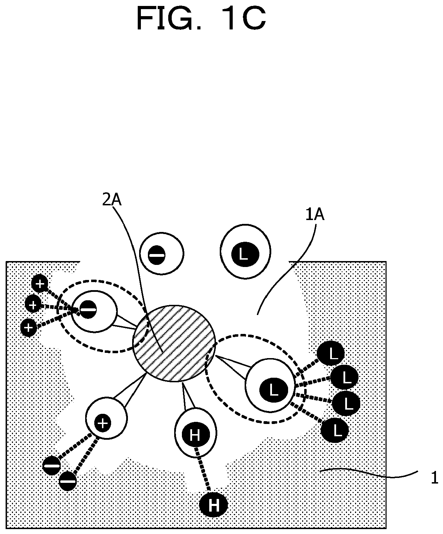

Each atom or each group of atoms moves depending on interactions within the binding site 1A during the molecular dynamics calculation. During that time, atoms or groups of atoms satisfying the conditions for binding to the base fragment molecule 2 bind to the base fragment molecule 2, to form a grown base fragment molecule 2A (FIG. 1C).

As a result, an appropriate ligand (a grown molecule) that takes into consideration changes in the three-dimensional structures of the target molecule 1 and the binding site 1A is obtained.

### <Growth Step>

In the growth step, molecular dynamics calculation using a reactive force field is performed, and atoms are bonded to the base fragment molecule at the binding site of the target molecule, so that the base fragment molecule is grown, and a grown molecule is obtained.

The molecular dynamics calculation can be performed according to a molecular dynamics calculation program. Examples of the molecular dynamics calculation program include AMBER, CHARMm, GROMACS, GROMOS, NAMD, and myPresto, for example.

A reactive force field is a force field in which bond generation and cleavage can be written, and various parameters have been reported in the following papers and the like, for example.
- J. Phys. Chem. A 2001, 105, 9396-9409
- J. Phys. Chem. B 2011, 115, 249-261
- Phys. Chem. Chem. Phys., 2013, 15, 15062-15077

An example of the reactive force field is ReaxFF introduced in the above papers, for example.

There are no particular restrictions on the target molecule, and any appropriate molecule may be selected as the target molecule. For example, the target molecule may be protein, ribonucleic acid (RNA), deoxyribonucleic acid (DNA), or the like.

There are no particular restrictions on the time for the molecular dynamics calculation, and any appropriate time can be selected according to the purpose.

There are no particular restrictions on the base fragment molecule, and any appropriate molecule can be selected according to the purpose, as long as the molecule has a ring structure. Examples of the ring structure include alicyclic hydrocarbons, aromatic hydrocarbons, and heterocyclic rings.

Since a ring structure is empirically known to interact strongly with the binding site of a target molecule, a ring structure is suitable as a base fragment from which a molecule is to be grown.

The bonding of atoms to the base fragment molecule in the growth step is performed on the basis of the distance and the angle between the base fragment molecule and each atom, for example. These can be set as appropriate in accordance with the parameters for a reactive force field.

The growth step includes a primary growth process and a secondary growth process, for example. In the growth step, the secondary growth process is repeated a plurality of times, for example, to further grow the molecule.

In the primary growth process, atoms are bonded to the base fragment molecule placed at the binding site of the target molecule, so that the base fragment molecule is grown. As a result, a grown primary molecule is obtained. This process is performed through molecular dynamics calculation using a reactive force field.

In the secondary growth process, atoms are bonded to the primary molecule placed at the binding site of the target molecule, so that the primary molecule is grown. As a result, a grown secondary molecule is obtained. This process is performed through molecular dynamics calculation using a reactive force field.

The simulation time for the molecular dynamics calculation in the primary growth process and the secondary growth process may be several nanoseconds to several tens of nanoseconds (for example, about 1 nanosecond to 50 nanoseconds).

When the primary molecule obtained in the primary growth process is subjected to the secondary growth process, it is preferable to perform structure optimization on the primary molecule. The structure optimization may be performed through quantum chemical calculation, for example.

As the structure optimization is performed, a stable three-dimensional structure is obtained. As a result, the accuracy of the growth step becomes higher, and the evaluation based on the molecular force field of the interaction between the grown molecule obtained in the growth step and the target molecule becomes more accurate.

Further, when the primary molecule subjected to the structure optimization is subjected to the secondary growth process, it is preferable to perform structure relaxation on the complex of the target molecule and the primary molecule through molecular dynamics calculation using a molecular force field. As a result, the accuracy of the secondary growth process is increased. The molecular force field used herein is not limited to any particular one, and an appropriate molecular force field can be selected according to the purpose. For example, the molecular force field used herein may be a molecular force field accompanying a molecular dynamics calculation program, such as AMBER, CHARMm, GROMACS, GROMOS, NAMD, or myPresto, for example.

Note that, when the secondary molecule obtained in the secondary growth process is further subjected to a secondary growth process, it is preferable to perform structure optimization on the secondary molecule.

Further, when the secondary molecule subjected to the structure optimization is further subjected to a secondary growth process, it is preferable to perform structure relaxation on the complex of the target molecule and the secondary molecule through molecular dynamics calculation using a molecular force field.

### < Extraction Step>

In the extraction step, a candidate compound that is a candidate for a compound is extracted with the use of an appearance frequency and a molecule prepared with an atom density distribution.

The appearance frequency is the appearance frequency in the step of growth of each of the chemical structures of grown molecules obtained in a plurality of growth steps.

The molecule prepared with the use of an atom density distribution is a molecule prepared with the use of an atom density distribution obtained by superposing a plurality of grown molecules obtained in a plurality of growth steps.

A molecule having a higher appearance frequency is more likely to be a compound having a strong interaction with the target molecule.

Further, even in the case of a molecule with a low appearance frequency in the growth step or a molecule that does not appear in the growth step, there is a high possibility that a molecule prepared with the use of an atom density distribution obtained by superimposing a plurality of grown molecules obtained in a plurality of growth steps is a compound having a strong interaction with the target molecule.

This is because, in some cases, there exists a bond that is not generated in the bond generation using a reactive force field, but is preferably generated when the atom density distribution is taken into consideration. For example, in the generation of a bond using a reactive force field, the probability of generation of a ring structure is considered relatively low. Therefore, in a case where it is preferable to generate a ring structure when an atom density distribution is taken into consideration, it is preferable to prepare a molecule having a ring structure using the atom density distribution.

An example of the compound search method is now described, with reference to flowcharts and drawings.

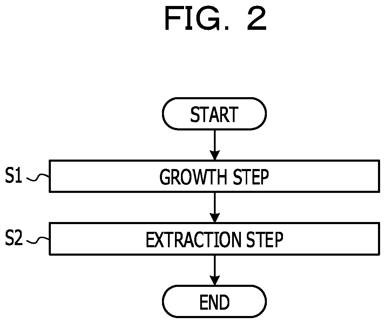

FIG. 2 shows a flowchart of an example of the compound search method.

First, a growth step is carried out (S1). In the growth step, molecular dynamics calculation using a reactive force field is performed, and atoms are bonded to the base fragment molecule at the binding site of the target molecule, so that the base fragment molecule is grown, and a grown molecule is obtained.

The extraction step is then carried out (S2). In the extraction step, a molecule created with the use of the appearance frequency in the step of growth of each of the chemical structures of a plurality of grown molecules obtained in a plurality of growth steps, and an atom density distribution obtained by superimposing the grown molecules obtained in the plurality of growth steps is used, to extract a candidate compound that is a candidate for a compound.

An example of the growth step is now described in detail, with reference to flowcharts and drawings.

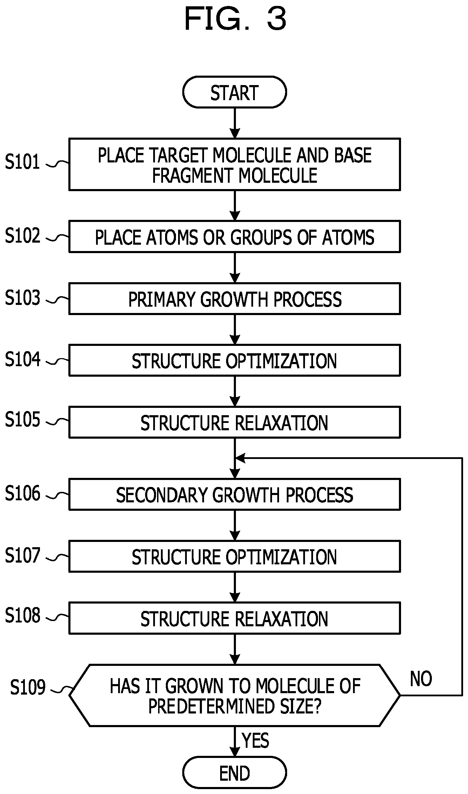

FIG. 3 shows a flowchart of an example of the growth step.

### < < Step S101 >>

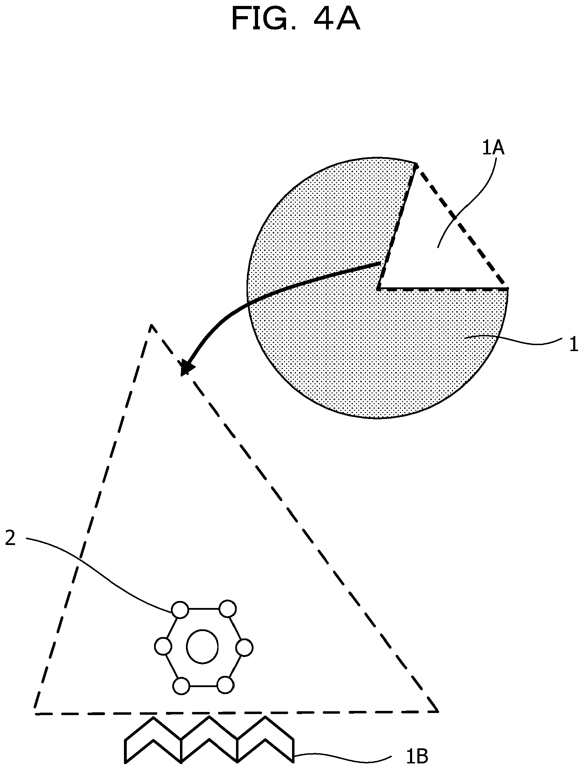

First, the target molecule 1 and the base fragment molecule 2 are placed (FIG. 4A, S101). In this step, the base fragment molecule 2 is placed at the binding site 1A of the target molecule 1. These placements are performed by constructing the three-dimensional structure of the target molecule and the three-dimensional structure of the base fragment molecule in a three-dimensional coordinate space, for example. The three-dimensional structure of the target molecule is a known three-dimensional structure, for example.

The position of the base fragment molecule 2 may be a position in the vicinity of amino-acid residues 1B that are amino-acid residues in the binding site 1A of the target molecule 1 and interact with the base fragment molecule 2, for example. As the base fragment molecule 2 is placed in the vicinity of such amino-acid residues, the interaction can be expected to be maintained in the molecular dynamics calculation.

Note that, as the initial structure in the molecular dynamics calculation, hydrogen atoms are taken into consideration in the target molecule 1, for example, but hydrogen atoms are not contained in the base fragment molecule 2. Therefore, the target molecule 1 to be placed contains hydrogen atoms, but the base fragment molecule 2 does not contain hydrogen atoms.

The three-dimensional structure data for forming the three-dimensional structure of the target molecule and the three-dimensional structure of the base fragment molecule includes atom information data, coordinate information data, and bond information data, for example.

The format of these pieces of data is not limited to any particular format, and may be appropriately selected according to the purpose. For example, the format may be text data, the Structure Data File (SDF) format, or the MOL file format.

### < < Step S102 >>

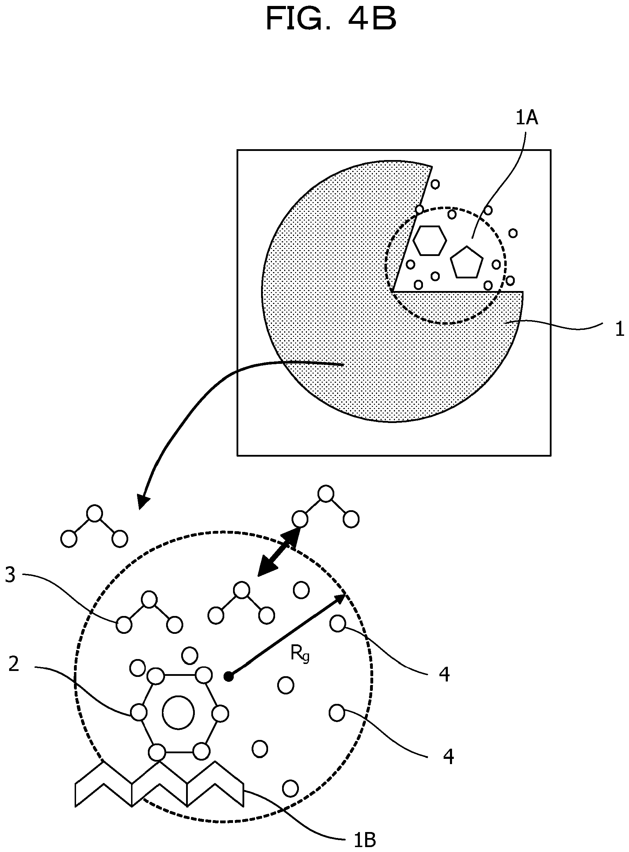

Next, atoms or groups of atoms 4 are placed in and around the binding site 1A (FIG. 4B, S102). In this step, water molecules 3 are also placed normally. The density of the water molecules may be approximately the same as the density of the water molecules placed according to a general molecular dynamics calculation. The number and the positions of the atoms or groups of atoms are not limited to any particular ones, and may be appropriately selected according to the purpose. For example, the number of the atoms or groups of atoms may be almost the same as the number of water molecules. The positions of the atoms or groups of atoms may be selected as appropriate, for example.

In a general molecular dynamics calculation, water molecules are placed at a density of about 997 kg/m³ (0.9% NaCl).

Each group of atoms to be placed may be a group of two to ten bonded atoms, for example. Such a group of atoms may be a functional group, for example.

Examples of the atoms or groups of atoms include carbon, nitrogen, oxygen, phosphorus, and halogen atoms.

The ratio of the respective elements in the atoms or groups of atoms to be placed is not limited to any particular ratio, and may be appropriately selected according to the purpose. For example, the ratio may be appropriately selected, with reference to the ratio of the respective elements in a known drug.

Further, predetermined constraints may be put on the water molecules and the atoms or groups of atoms so that the water molecules and the atoms or groups of atoms will not move farther away from the binding site 1A than necessary. The constraints are applied in a spherical space with a radius Ra in the binding site 1A, for example.

### << Step S103 >>

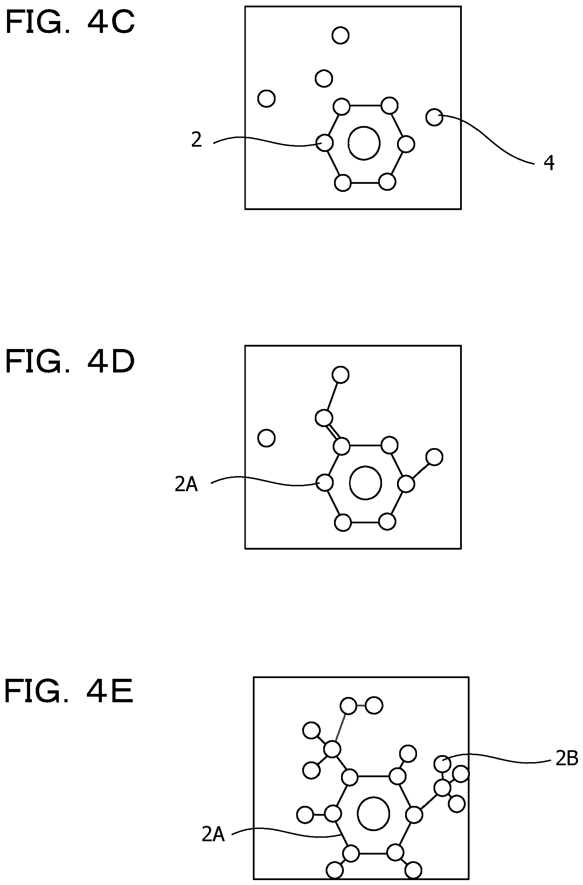

Next, molecular dynamics calculation using a reactive force field is then performed as the primary growth process (S103). For example, new bonds are generated through the molecular dynamics calculation using a reactive force field, so that the grown base fragment molecule 2A (a grown primary molecule molecule) illustrated in FIG. 4D is obtained from the base fragment molecule 2 and the atoms 4 illustrated in FIG. 4C. A new bond is generated when an atom of the base fragment molecule 2 and an atom 4 are at a predetermined distance and form a predetermined angle, for example.

The predetermined distance and the predetermined angle are appropriately set in accordance with the type of an atom to be bonded, the type of the other atom to be bonded to the atom, the type of the bond to be formed with the atom, and the like, for example.

Further, in the primary growth process, cleavage is preferably not caused in the bonds in the base fragment molecule 2A.

### < < Step S104 > >

Next, structure optimization is performed on the primary molecule (the grown base fragment molecule 2A) obtained in the primary growth process (S104). At the time of the structure optimization, hydrogen atoms 2B are first added to the primary molecule so that the primary molecule has a structurally consistent chemical structure (FIG. 4E).

Structure optimization is then performed on the primary molecule having the hydrogen atoms 2B added thereto. As the structure optimization is performed, a stable three-dimensional structure is obtained for the primary molecule. The structure optimization is performed through quantum chemical calculation, for example.

### < < Step S105 > >

Next, before the primary molecule (the grown base fragment molecule 2A) subjected to the structure optimization is subjected to a secondary growth process, structure relaxation is performed on the complex of the target molecule and the primary molecule through molecular dynamics calculation using a molecular force field (S105).

### < < Step S106 >>

Next, the primary molecule, and atoms or groups of atoms are placed at the binding site of the target molecule, and molecular dynamics calculation using a reactive force field is performed as a secondary growth process (S106). As a result, new bonds are generated, so that the primary molecule is grown, and a grown secondary molecule is obtained.

Note that, in the secondary growth process, it is preferable not to cause cleavage at the bonds in the primary molecule.

### < < Step S107 >>

Next, structure optimization is performed on the secondary molecule obtained in the secondary growth process (S107). At the time of the structure optimization, hydrogen is first added to the secondary molecule, so that the secondary molecule has a structurally consistent chemical structure.

Structure optimization is then performed on the secondary molecule having the hydrogen added thereto. As the structure optimization is performed, a stable three-dimensional structure is obtained. The structure optimization is performed through quantum chemical calculation, for example.

### < < Step S108 >>

Next, structure relaxation of the complex of the target molecule and the secondary molecule is performed through molecular dynamics calculation using a molecular force field (S108).

### < < Step S109 >>

In a case where the secondary molecule in step S108 is sufficiently small with respect to the size of the binding site, the molecule is preferably further grown, to search for more diverse compound structures.

Therefore, a check is made to determine whether the secondary molecule in step S108 has grown into a molecule of a predetermined size. In this determination, molecular weight is used as a criterion for determining whether the molecule has the predetermined size, for example.

If the result of the determination shows that the secondary molecule has not grown into a molecule of the predetermined size, a series of the processes, which are the secondary growth process (step S106), the structure optimization (step S107), and the structure relaxation (step S108), is repeated until the secondary molecule grows to a predetermined size.

If the secondary molecule has grown into a molecule of the predetermined size, on the other hand, the growth step is ended.

As a result of completion of the growth step, the following data is obtained, for example.
- Base fragment molecule growth history
- The complex structure of the target molecule and the secondary molecule

The growth history is obtained as a set of the structures of the primary molecule and the secondary molecule at the respective ends of the primary growth process and the secondary growth process, for example.

These sets of data are used in the extraction step.

Note that the growth step is performed a plurality of times, and the initial conditions for molecular dynamics calculation, such as the arrangement and the number of atoms or groups of atoms, are changed for each time the growth step is performed.

An example of the extraction step is now described in detail, with reference to flowcharts and drawings.

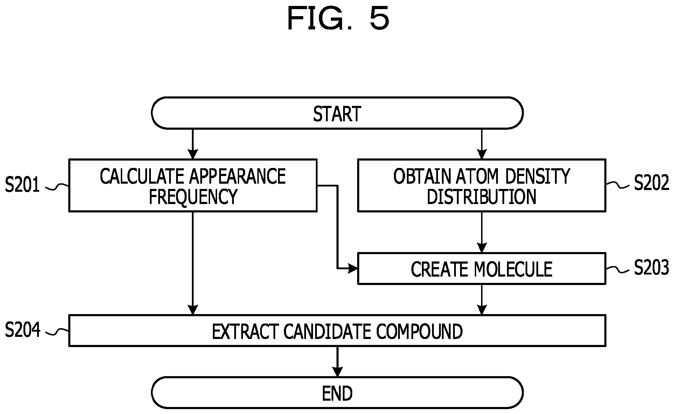

FIG. 5 shows a flowchart of an example of the extraction step.

In the extraction step, a candidate compound that is a candidate for a compound having a strong interaction with the target molecule is extracted on the basis of the data obtained in the growth step. The procedures are as follows, for example.

### < < Step S201 > >

First, the appearance frequency in the step of growth of each of the chemical structures of grown molecules obtained in a plurality of growth steps is calculated (S201).

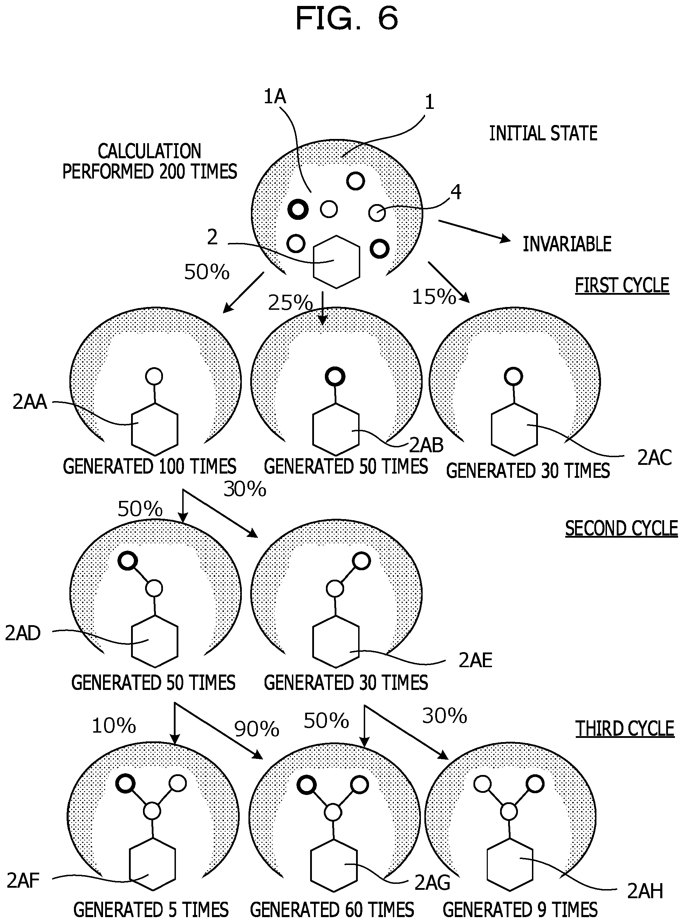

The appearance frequency may be represented by the number of times each grown molecule has been generated, as illustrated in FIG. 6, for example.

For example, the growth step is carried out 200 times. During that time, a growth process (a primary growth process or a secondary growth process) is performed up to three times (a first cycle, a second cycle, and a third cycle). Note that the first cycle corresponds to a primary growth process, the second cycle corresponds to a secondary growth process, and the third cycle corresponds to a secondary growth process.

For example, the primary growth process (the first cycle) for bonding atoms to the base fragment molecule 2 at the binding site 1A of the target molecule 1 is performed 200 times. As a result, as illustrated in FIG. 6, three kinds of grown molecules (2AA, 2AB, and 2AC) are generated, and the generation probabilities are 50%, 25%, and 15%, respectively. In that case, the numbers of times generation is performed are 100 times for 2AA, 50 times for 2AB, and 30 times for 2AC.

Next, the second cycle is performed on the complex of the molecule (2AA) generated 100 times and the target molecule 1. As a result, two kinds of grown molecules (2AD and 2AE) are generated as illustrated in FIG. 6. In a case where the generation probabilities thereof are 50% and 30%, respectively, the numbers of times generation is performed are 50 times for 2AD and 30 times for 2AE.

Further, the third cycle is performed on each of the complex of the molecule (2AD) generated 50 times and the target molecule 1, and the complex of the molecule (2AE) generated 30 times and the target molecule 1. As a result, three kinds of grown molecules (2AF, 2AG, and 2AH) are generated as illustrated in FIG. 6. In a case where the probability of generation of 2AF from 2AD is 10%, the probability of generation of 2AG from 2AD is 90%, the probability of generation of 2AG from 2AE is 50%, and the probability of generation of 2AH from 2AE is 30%, the numbers of times generation is performed are five times for 2AF, 60 times for 2AG, and nine times for 2AH.

The appearance frequencies may be shown for the respective cycles, or may be shown collectively.

Further, the appearance frequency may be shown according to the size of the generated molecule. The size of a molecule may be molecular weight, for example. For example, a molecule within a specific molecular weight range may be extracted, and the appearance frequency in the range may be shown as a result.

### < < Step S202 > >

Meanwhile, an atom density distribution formed by superimposing grown molecules obtained in a plurality of growth steps is obtained (S202).

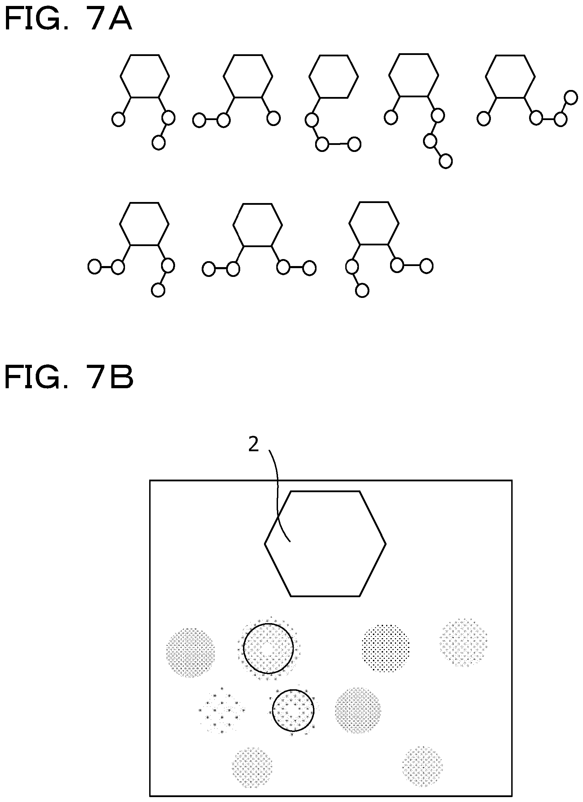

For example, in a case where a plurality of grown molecules as illustrated in FIG. 7A is obtained as a result of the growth step, these molecules are superimposed, to calculate an atom density distribution. The superposition is performed so that the base fragment molecules overlap, for example.

As a result, a density distribution of the atoms bonded to the base fragment molecule is obtained, as illustrated in FIG. 7B. Note that, in FIG. 7B, differences in the density of the atoms is represented by the degrees of darkness of circles.

### < < Step S203 > >

Next, a molecule is created from the obtained atom density distribution (S203).

The molecule is preferably created so that a ring structure is generated.

Further, in the production of the molecule, the appearance frequency information obtained in step S201 is preferably taken into consideration.

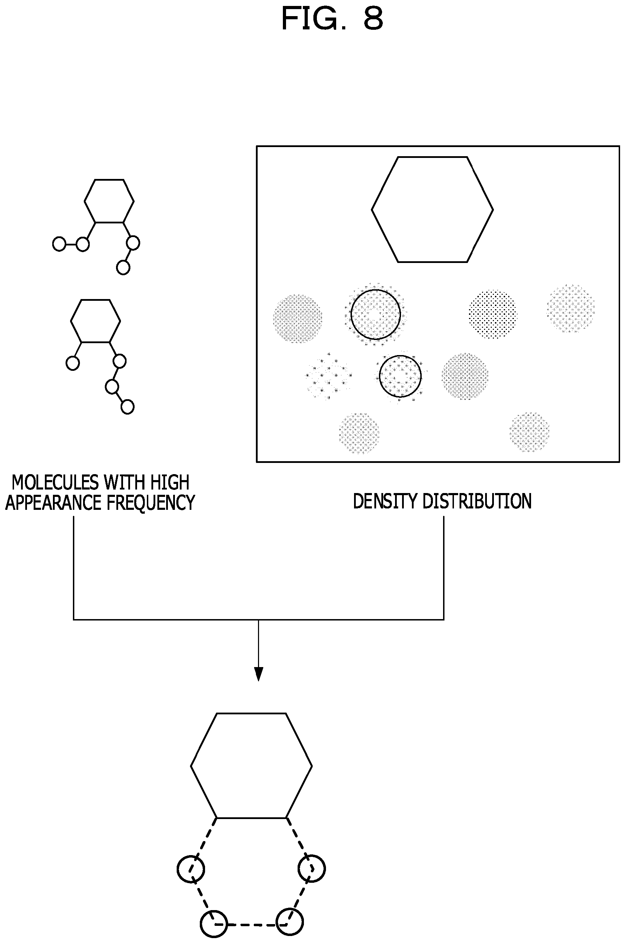

For example, as illustrated in FIG. 8, a molecule that has not been obtained in the growth step and has a ring structure is created in addition to the base fragment molecule, with a high-appearance-frequency molecule and a density distribution being taken into consideration.

### < < Step S204 > >

Next, a candidate compound that is a candidate for a compound having a strong interaction with the target molecule is extracted, on the basis of the appearance frequency and the created molecule (S204).

The extraction is performed by outputting a high-appearance-frequency molecule and the molecule created in step S203, for example.

An example of the extraction step is now introduced.

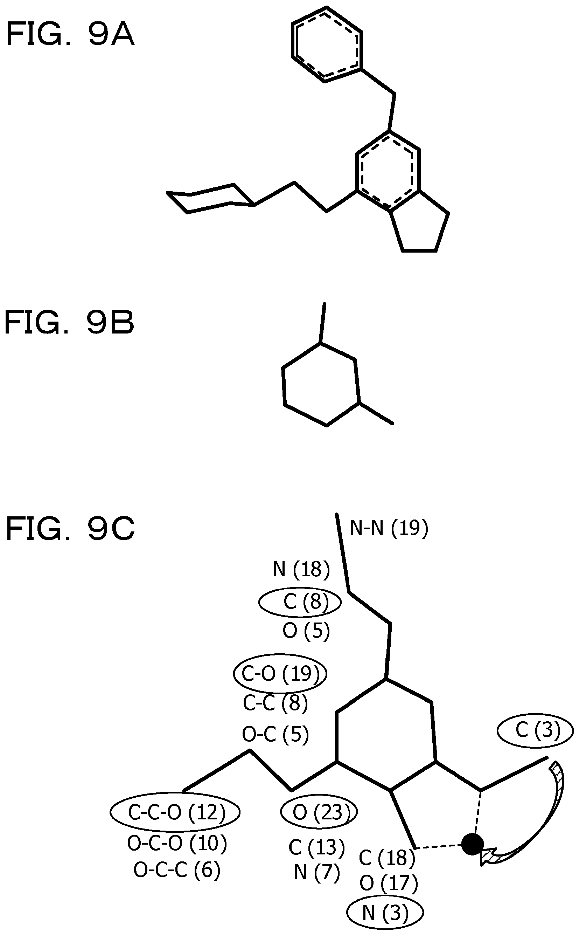

FIG. 9A illustrates the structure of a compound actually included in an X-ray crystal structure.

This structure was searched for through the growth step of the disclosed compound search method. Specifically, for example, a three-dimensional structure of cyclin-dependent kinase 2 (CDK2) (PDB ID: 1H1Q) was used as the protein, the structure illustrated in FIG. 9B was selected as the base fragment molecule, and the growth step was performed 50 times. As a result, the structure illustrated in FIG. 9C was obtained. In this structure, a symbol such as C-O (19) indicates an atom attached to the base fragment molecule and the number of times the atom has been generated. The structure illustrated in FIG. 9C included a structure similar to the compound illustrated in FIG. 9A.

### (Program)

A compound search program disclosed herein is a program for causing a computer to implement the disclosed compound search method.

In the compound search program, a preferred mode in implement of the compound search method is the same as a preferred mode in the disclosed compound search method.

The compound search program can be created by using various kinds of known program languages, depending on the configuration of the computer system to be used and the type/version of the operating system.

The program may be recorded on a recording medium such as an internal hard disk or an external hard disk, or may be recorded on a recording medium such as a compact disc read only memory (CD-ROM), a digital versatile disk read only memory (DVD-ROM), a magneto-optical (MO) disk, or a universal serial bus (USB) memory [USB flash drive], for example. In a case where the program is recorded on a recording medium such as a CD-ROM, a DVD-ROM, an MO disk, or a USB memory, the program can be directly used through a recording medium reader included in the computer system, or be installed into a hard disk and be then used, as needed. Alternatively, the program may be recorded in an external storage area (another computer or the like) that is accessible from the computer system through an information communication network, and this program may be directly used from the external storage area through an information communication network, or be installed into a hard disk and then be used, as needed.

The program may be divided into respective processes, and be recorded on a plurality of recording media.

### (Computer-Readable Recording Medium)

A computer-readable recording medium disclosed herein records the disclosed program.

The computer-readable recording medium is not limited to any particular medium, and may be appropriately selected according to the purpose. For example, the computer-readable recording medium may be an internal hard disk, an external hard disk, a CD-ROM, a DVD-ROM, an MO disk, a USB memory, or the like.

The recording medium may be a plurality of recording media on which the program that is divided into respective processes is recorded.

### (Compound Search Device)

A compound search device disclosed herein includes a growing unit, for example.

The disclosed compound search device includes an extracting unit, for example.

The growing unit carries out the growth step.

The extracting unit carries out the extraction step.

A preferred mode of a processing method at each unit in the compound search device is the same as a preferred mode of each step in the disclosed compound search method.

The compound search device may be a plurality of compound search devices including a plurality of recording media on which the respective processes of a divided program are recorded.

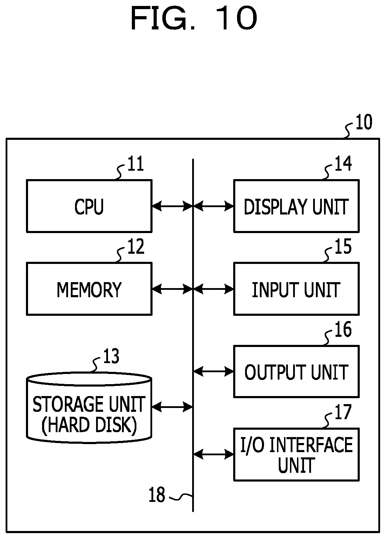

FIG. 10 illustrates an example of the disclosed compound search device.

A compound search device 10 is formed with a CPU 11, a memory 12, a storage unit 13, a display unit 14, an input unit 15, an output unit 16, an I/O interface unit 17, and the like that are connected via a system bus 18, for example.

The CPU (Central Processing Unit) 11 performs arithmetic operations (such as the four arithmetic operations and comparison operations), hardware and software operation control, and the like.

The memory 12 is a memory including a random access memory (RAM) and a read only memory (ROM), for example. The RAM stores an operating system (OS) and an application program read from the ROM and the storage unit 13, and functions as a main memory and a work area of the CPU 11.

The storage unit 13 is a device that stores various kinds of programs and data, and may be a hard disk, for example. The storage unit 13 stores a program to be executed by the CPU 11, the data to be used in executing the program, the OS, and the like.

The program is stored in the storage unit 13, is loaded into the RAM (the main memory) of the memory 12, and is executed by the CPU 11.

The display unit 14 is a display device, and may be a display device such as a CRT monitor or a liquid crystal panel, for example.

The input unit 15 is an input device for various kinds of data, and may be a keyboard, a pointing device (such as a mouse), or the like, for example.

The output unit 16 is an output device for various kinds of data, and may be a printer, for example.

The I/O interface unit 17 is an interface for connecting various external devices. For example, the I/O interface unit 17 enables inputting/outputting of data into/from a CD-ROM, a DVD-ROM, an MO disk, a USB memory, or the like.

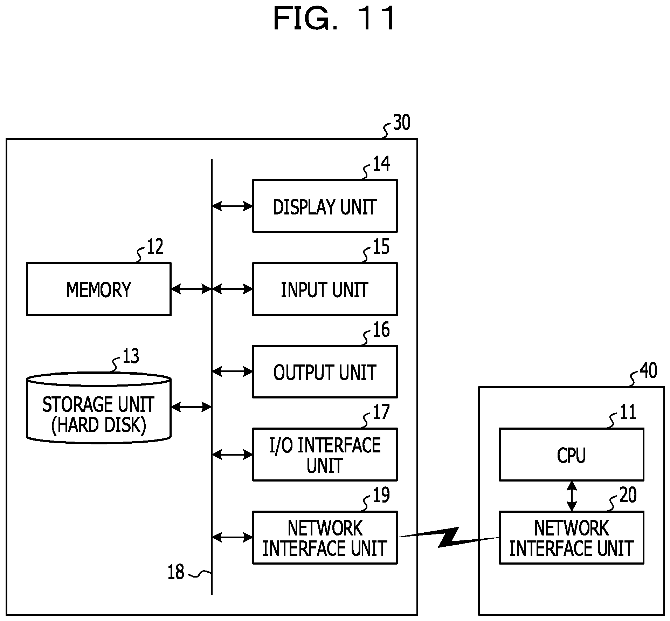

FIG. 11 illustrates another example of the disclosed compound search device.

The example illustrated in FIG. 11 is a cloud-type configuration example, and the CPU 11 is independent of the storage unit 13 and the like. In this configuration example, a computer 30 that includes the storage unit 13 and the like, and a computer 40 that includes the CPU 11 are connected via network interface units 19 and 20.

The network interface units 19 and 20 are hardware that performs communication using the Internet.

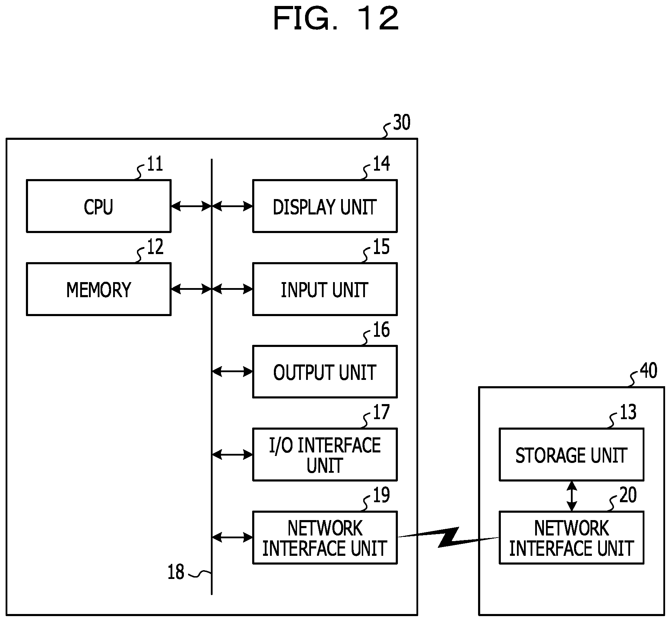

FIG. 12 illustrates yet another example of the disclosed compound search device.

The example illustrated in FIG. 12 is a cloud-type configuration example, and the storage unit 13 is independent of the CPU 11 and the like. In this configuration example, a computer 30 that includes the CPU 11 and the like, and a computer 40 that includes the storage unit 13 are connected via network interface units 19 and 20.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

## Claims

1. A computer-implemented method for searching for a compound having a strong interaction with a target molecule, the method comprising
growing a base fragment molecule and obtaining a grown molecule by performing molecular dynamics calculation using a reactive force field and bonding an atom to the base fragment molecule at a binding site of the target molecule wherein the growing includes the steps of:
a primary growth process for growing the base fragment molecule and obtaining a grown primary molecule, by bonding an atom to the base fragment molecule located at the binding site of the target molecule; and
a secondary growth process for growing the primary molecule and obtaining a grown secondary molecule, by bonding an atom to the primary molecule located at the binding site of the target molecule,
wherein the method further includes
extracting a candidate compound that is a candidate for the compound, using a molecule created by the said growing with
an appearance frequency obtained by creating a plurality of grown molecules obtained from the said growing steps, wherein the appearance frequency corresponds to a number of times each grown molecule has been obtained; and
an atom density distribution formed by superimposing the grown molecules obtained in the plurality of the said growing steps

2. The computer-implemented method for searching for a compound according to claim 1, wherein, in the growing, the atom is bonded to the base fragment molecule on a basis of a distance and an angle between the base fragment molecule and the atom.

3. A compound search device that searches for a compound having a strong interaction with a target molecule,
the compound search device comprising
a growing unit that performs growing to grow a base fragment molecule and obtain a grown molecule, by performing molecular dynamics calculation using a reactive force field and bonding an atom to the base fragment molecule at a binding site of the target molecule, wherein the growing includes the steps of:
a primary growth process for growing the base fragment molecule and obtaining a grown primary molecule, by bonding an atom to the base fragment molecule located at the binding site of the target molecule; and
a secondary growth process for growing the primary molecule and obtaining a grown secondary molecule, by bonding an atom to the primary molecule located at the binding site of the target molecule,
wherein the compound search device further incudes
an extracting unit that performs extracting of a candidate compound that is a candidate for the compound, using a molecule created by the said growing with
an appearance frequency obtained by creating a plurality of grown molecules obtained from the said growing steps, wherein the appearance frequency corresponds to a number of times each grown molecule has been obtained; and
an atom density distribution formed by superimposing the grown molecules obtained in the plurality of the said growing steps.

4. The compound search device according to claim 3, wherein, in the growing, the atom is bonded to the base fragment molecule on a basis of a distance and an angle between the base fragment molecule and the atom.

5. A program for causing a computer to execute a compound search process for searching for a compound having a strong interaction with a target molecule,
the compound search process comprising
growing a base fragment molecule and obtain a grown molecule, by performing molecular dynamics calculation using a reactive force field and bonding an atom to the base fragment molecule at a binding site of the target molecule, wherein the growing includes the steps of:
a primary growth process for growing the base fragment molecule and obtaining a grown primary molecule, by bonding an atom to the base fragment molecule located at the binding site of the target molecule; and
a secondary growth process for growing the primary molecule and obtaining a grown secondary molecule, by bonding an atom to the primary molecule located at the binding site of the target molecule,
wherein the compound search program further includes
extracting a candidate compound that is a candidate for the compound, using a molecule created by the said growing with
an appearance frequency obtained by creating a plurality of grown molecules obtained from the said growing steps, wherein the appearance frequency corresponds to a number of times each grown molecule has been obtained; and
an atom density distribution formed by superimposing the grown molecules obtained in the plurality of the said growing steps.

6. The program according to claim 5, wherein, in the growing, the atom is bonded to the base fragment molecule on a basis of a distance and an angle between the base fragment molecule and the atom.

## Patentansprüche

1. Computerimplementiertes Verfahren zum Suchen nach einer Verbindung mit einer starken Wechselwirkung mit einem Zielmolekül, wobei das Verfahren Folgendes umfasst:
Züchten eines Basisfragmentmoleküls und Erlangen eines gezüchteten Moleküls durch Durchführen einer Molekulardynamikberechnung unter Verwendung eines reaktiven Kraftfelds und Binden eines Atoms an das Basisfragmentmolekül an einer Bindungsstelle des Zielmoleküls, wobei das Züchten die folgenden Schritte beinhaltet:
einen primären Züchtungsprozess zum Züchten des Basisfragmentmoleküls und Erlangen eines gezüchteten Primärmoleküls durch Binden eines Atoms an das Basisfragmentmolekül, das sich an der Bindungsstelle des Zielmoleküls befindet; und
einen sekundären Züchtungsprozess zum Züchten des Primärmoleküls und Erlangen eines gezüchteten Sekundärmoleküls durch Binden eines Atoms an das Primärmolekül, das sich an der Bindungsstelle des Zielmoleküls befindet,
wobei das Verfahren ferner Folgendes beinhaltet:
Extrahieren einer Kandidatenverbindung, die ein Kandidat für die Verbindung ist, unter Verwendung eines Moleküls, das durch das Züchten
mit einer Auftrittshäufigkeit erzeugt wurde, die durch Erzeugen einer Vielzahl von gezüchteten Molekülen aus den Züchtungsschritten erlangt wird, wobei die Auftrittshäufigkeit der Häufigkeit entspricht, mit der jedes gezüchtete Molekül erlangt wurde; und
eine Atomdichteverteilung, die durch Überlagern der in der Vielzahl von Züchtungsschritten erlangten gezüchteten Moleküle gebildet wird.

2. Computerimplementiertes Verfahren zum Suchen nach einer Verbindung nach Anspruch 1, wobei das Atom beim Züchten auf Grundlage eines Abstands und eines Winkels zwischen dem Basisfragmentmolekül und dem Atom an das Basisfragmentmolekül gebunden wird.

3. Verbindungssuchvorrichtung, das nach einer Verbindung sucht, die eine starke Wechselwirkung mit einem Zielmolekül aufweist, wobei die Verbindungssuchvorrichtung Folgendes umfasst:
eine Züchtungseinheit, die eine Züchtung zum Züchten eines Basisfragmentmoleküls und Erlangen eines gezüchteten Moleküls durch Durchführen einer Molekulardynamikberechnung unter Verwendung eines reaktiven Kraftfelds und Binden eines Atoms an das Basisfragmentmolekül an einer Bindungsstelle des Zielmoleküls durchführt, wobei das Züchten die folgenden Schritte beinhaltet:
einen primären Züchtungsprozess zum Züchten des Basisfragmentmoleküls und Erlangen eines gezüchteten Primärmoleküls durch Binden eines Atoms an das Basisfragmentmolekül, das sich an der Bindungsstelle des Zielmoleküls befindet; und
einen sekundären Züchtungsprozess zum Züchten des Primärmoleküls und Erlangen eines gezüchteten Sekundärmoleküls durch Binden eines Atoms an das Primärmolekül, das sich an der Bindungsstelle des Zielmoleküls befindet,
wobei die Verbindungssuchvorrichtung ferner Folgendes beinhaltet:
eine Extrahierungseinheit, die Extrahieren einer Kandidatenverbindung, die ein Kandidat für die Verbindung ist, unter Verwendung eines Moleküls durchführt, das durch das Züchten
mit einer Auftrittshäufigkeit erzeugt wurde, die durch Erzeugen einer Vielzahl von gezüchteten Molekülen aus den Züchtungsschritten erlangt wird, wobei die Auftrittshäufigkeit der Häufigkeit entspricht, mit der jedes gezüchtete Molekül erlangt wurde; und
eine Atomdichteverteilung, die durch Überlagern der in der Vielzahl von Züchtungsschritten erlangten gezüchteten Moleküle gebildet ist.

4. Verbindungssuchvorrichtung nach Anspruch 3, wobei das Atom beim Züchten auf Grundlage eines Abstands und eines Winkels zwischen dem Basisfragmentmolekül und dem Atom an das Basisfragmentmolekül gebunden wird.

5. Programm, um einen Computer zu veranlassen, einen Verbindungssuchvorgang auszuführen, um nach einer Verbindung zu suchen, die eine starke Wechselwirkung mit einem Zielmolekül aufweist,
wobei der Verbindungssuchvorgang Folgendes umfasst:
Züchten eines Basisfragmentmoleküls und Erlangen eines gezüchteten Moleküls durch Durchführen einer Molekulardynamikberechnung unter Verwendung eines reaktiven Kraftfelds und Binden eines Atoms an das Basisfragmentmolekül an einer Bindungsstelle des Zielmoleküls, wobei das Züchten die folgenden Schritte beinhaltet:
einen primären Züchtungsprozess zum Züchten des Basisfragmentmoleküls und Erlangen eines gezüchteten Primärmoleküls durch Binden eines Atoms an das Basisfragmentmolekül, das sich an der Bindungsstelle des Zielmoleküls befindet; und
einen sekundären Züchtungsprozess zum Züchten des Primärmoleküls und zum Erhalten eines gewachsenen Sekundärmoleküls durch Binden eines Atoms an das Primärmolekül, das sich an der Bindungsstelle des Zielmoleküls befindet,
wobei das Verbindungssuchprogramm ferner Folgendes beinhaltet: Extrahieren einer Kandidatenverbindung, die ein Kandidat für die Verbindung ist, unter Verwendung eines Moleküls, das durch das Züchten
mit einer Auftrittshäufigkeit erzeugt wurde, die durch Erzeugen einer Vielzahl von gezüchteten Molekülen aus den Züchtungsschritten erlangt wird, wobei die Auftrittshäufigkeit der Häufigkeit entspricht, mit der jedes gezüchtete Molekül erlangt wurde; und
eine Atomdichteverteilung, die durch Überlagern der in der Vielzahl von Züchtungsschritten erlangten gezüchteten Moleküle gebildet ist.

6. Programm nach Anspruch 5, wobei das Atom beim Züchten auf Grundlage eines Abstands und eines Winkels zwischen dem Basisfragmentmolekül und dem Atom an das Basisfragmentmolekül gebunden wird.

## Revendications

1. Procédé mis en œuvre par ordinateur de recherche d'un composé ayant une forte interaction avec une molécule cible, le procédé comprenant
la croissance d'une molécule de fragment de base et l'obtention d'une molécule développée en effectuant un calcul de dynamique moléculaire à l'aide d'un champ de force réactif et en liant un atome à la molécule de fragment de base au niveau d'un site de liaison de la molécule cible, dans lequel la croissance comporte les étapes :
d'un processus de croissance primaire pour faire croître la molécule de fragment de base et obtenir une molécule primaire développée, en liant un atome à la molécule de fragment de base située au niveau du site de liaison de la molécule cible ; et
d'un processus de croissance secondaire pour faire croître la molécule primaire et obtenir une molécule secondaire développée, en liant un atome à la molécule primaire située au niveau du site de liaison de la molécule cible,
dans lequel le procédé comporte en outre
l'extraction d'un composé candidat qui est un candidat pour le composé, à l'aide d'une molécule créée par ladite croissance avec
une fréquence d'apparition obtenue en créant une pluralité de molécules développées obtenues à partir desdites étapes de croissance, dans lequel la fréquence d'apparition correspond à un nombre de fois où chaque molécule développée a été obtenue ; et
une distribution de densité atomique formée en superposant les molécules développées obtenues dans la pluralité desdites étapes de croissance.

2. Procédé mis en œuvre par ordinateur de recherche d'un composé selon la revendication 1, dans lequel, lors de la croissance, l'atome est lié à la molécule de fragment de base sur la base d'une distance et d'un angle entre la molécule de fragment de base et l'atome.

3. Dispositif de recherche de composés qui recherche un composé ayant une forte interaction avec une molécule cible,
le dispositif de recherche de composés comprenant
une unité de croissance qui réalise une croissance pour faire croître une molécule de fragment de base et obtenir une molécule développée en effectuant un calcul de dynamique moléculaire à l'aide d'un champ de force réactif et en liant un atome à la molécule de fragment de base au niveau d'un site de liaison de la molécule cible, dans lequel la croissance comprend les étapes :
d'un processus de croissance primaire pour faire croître la molécule de fragment de base et obtenir une molécule primaire développée, en liant un atome à la molécule de fragment de base située au niveau du site de liaison de la molécule cible ; et
d'un processus de croissance secondaire pour faire croître la molécule primaire et obtenir une molécule secondaire développée, en liant un atome à la molécule primaire située au niveau du site de liaison de la molécule cible,
dans lequel le dispositif de recherche de composés comporte en outre
une unité d'extraction qui effectue l'extraction d'un composé candidat qui est un candidat pour le composé, à l'aide d'une molécule créée par ladite croissance avec
une fréquence d'apparition obtenue en créant une pluralité de molécules développées obtenues à partir desdites étapes de croissance, dans lequel la fréquence d'apparition correspond à un nombre de fois où chaque molécule développée a été obtenue ; et
une distribution de densité atomique formée en superposant les molécules développées obtenues dans la pluralité desdites étapes de croissance.

4. Dispositif de recherche de composés selon la revendication 3, dans lequel, lors de la croissance, l'atome est lié à la molécule de fragment de base sur la base d'une distance et d'un angle entre la molécule de fragment de base et l'atome.

5. Programme pour amener un ordinateur à exécuter un processus de recherche de composés pour rechercher un composé ayant une forte interaction avec une molécule cible,
le processus de recherche de composés comprenant
la croissance d'une molécule de fragment de base et l'obtention d'une molécule développée en effectuant un calcul de dynamique moléculaire à l'aide d'un champ de force réactif et en liant un atome à la molécule de fragment de base au niveau d'un site de liaison de la molécule cible, dans lequel la croissance comporte les étapes :
d'un processus de croissance primaire pour faire croître la molécule de fragment de base et obtenir une molécule primaire développée, en liant un atome à la molécule de fragment de base située au niveau du site de liaison de la molécule cible ; et
d'un processus de croissance secondaire pour faire croître la molécule primaire et obtenir une molécule secondaire développée, en liant un atome à la molécule primaire située au niveau du site de liaison de la molécule cible,
dans lequel le programme de recherche de composés comporte en outre
l'extraction d'un composé candidat qui est un candidat pour le composé, à l'aide d'une molécule créée par ladite croissance avec
une fréquence d'apparition obtenue en créant une pluralité de molécules développées obtenues à partir desdites étapes de croissance, dans lequel la fréquence d'apparition correspond à un nombre de fois où chaque molécule développée a été obtenue ;
et
une distribution de densité atomique formée en superposant les molécules développées obtenues dans la pluralité desdites étapes de croissance.

6. Programme selon la revendication 5, dans lequel, lors de la croissance, l'atome est lié à la molécule de fragment de base sur la base d'une distance et d'un angle entre la molécule de fragment de base et l'atome.
